# FASCICULE DE BREVET EUROPEEN

(11) **EP 2 643 692 B1**
(45) Date de publication et mention de la délivrance du brevet: **03.01.2024**
(21) Numéro de dépôt: 11802496.7
(22) Date de dépôt: 22.11.2011
(51) Int. Cl.: B65D 81/26, B65D 75/32, G01N 33/487

(54) **ARTICLE POUR L'ANALYSE BIOLOGIQUE**
ARTIKEL ZUR BIOLOGISCHEN ANALYSE
ARTICLE FOR BIOLOGICAL ANALYSIS

(30) Priorité: 25.11.2010 FR 1059712
(43) Date de publication de la demande: 02.10.2013
(73) Titulaire: bioMérieux, 69280 Marcy l'Etoile (FR)
(72) Inventeur: FARGE, Agathe, F-42800 Rive de Gier (FR); JANUEL, Denis, F-69005 Lyon (FR)
(74) Mandataire: bioMérieux PI Groupement mandataires
(86) Numéro de dépôt international: PCT/FR2011/052727
(87) Numéro de publication internationale: WO 2012/069758

(56) Documents cités:
- EP-A1- 1 754 971
- EP-A1- 1 803 659
- US-A- 3 540 579
- US-A- 4 150 744
- US-A- 4 739 881
- US-A- 5 962 333

## Description

La présente invention concerne, de façon générale, le domaine de l'analyse, par exemple l'analyse biologique. Plus spécifiquement, la présente invention concerne un emballage pour un dispositif d'analyse.

Dans le domaine de l'analyse, en particulier celui de l'analyse biologique, il est courant d'utiliser des dispositifs stériles à usage unique emballés dans des emballages individuels. C'est le cas par exemple des dispositifs de prélèvement sanguin, tels que seringues ou aiguilles, des dispositifs de prélèvement de surface tels que les écouvillons, des dispositifs d'ensemencement de milieux de cultures, tels que les oeses jetables. Il est également commun de trouver des réactifs emballés dans des emballages à usage unique.

La plupart du temps, les emballages de ces dispositifs sont constitués de deux films plastiques scellés entre eux sur leur pourtour, l'un des deux étant généralement transparent. Un des deux films peut également être un film métallique, tel qu'un film d'aluminium ou un film multicouches constitué par exemple d'une couche d'aluminium et d'une couche plastique.

L'ouverture de ce type d'emballage peut se faire de différentes manières. La première consiste à dissocier les deux films constituant l'emballage. A cette fin, une zone au niveau de laquelle les deux films ne sont pas scellés, généralement dans un angle, permet de saisir chacun d'eux afin de les dissocier. La manipulation de ce type d'emballage n'est pas toujours très aisée. En effet, l'étape de séparation des deux films peut parfois être délicate du fait de la préhension difficile des deux films. L'utilisation de gants de protection par le manipulateur rend cette étape encore plus délicate. Par ailleurs, une fois les deux films désolidarisés, il peut arriver que le dispositif d'analyse glisse de l'emballage et tombe. Il devient alors inutilisable.

Une deuxième manière pour ouvrir l'emballage consistent à utiliser le dispositif d'analyse, lorsque celui-ci est rigide pour percer un des deux films constituant l'emballage. Il est évident qu'au moins un des films doit présenter une résistance à la rupture limitée pour permettre au dispositif d'analyse de le percer. A cette fin, le dispositif est pressé contre un des deux films de l'emballage. Un tel emballage est par exemple décrit dans le document US-3,036,700. Préférentiellement, le film décrit dans ce document et destiné à être percé, comporte une zone de faiblesse pour faciliter le percement. Un tel moyen d'ouverture nécessite que le dispositif d'analyse soit un dispositif rigide et suffisamment robuste pour supporter la mise sous contrainte.

Une troisième manière pour ouvrir l'emballage consiste à disposer à l'intérieur de l'emballage d'un objet contondant, ledit objet permettant de percer un des deux films constituant l'emballage. Un tel emballage est par exemple décrit dans le document US-4,739,881. Le principal problème constitué par un tel assemblage est le risque encouru par le manipulateur lors de l'ouverture de l'emballage. En effet, le manipulateur peut facilement se blesser avec l'objet contondant au moment où ce dernier perce l'emballage.

Le document EP174971 décrit une bandelette de diagnostic rapide comprenant un matériau dessicant. La bandelette comporte une base en plastique incorporant le matériau dessicant et est solidaire de la partie réactionnelle au moyen d'un adhésif double-face. La bandelette ainsi constituée est emballée dans une emballage blister dans lequel est logée la bandelette.

De plus le document EP1803659 concerne un emballage pour bandelettes de test réactionnel. L'emballage comporte une bandelette de test réactionnel, un agent dessicant et un conteneur comprenant une première chambre destinée à recevoir la bandelette test, une seconde chambre destinée à recevoir l'agent dessicant et un opercule pour sceller les première et seconde chambre.

Eu égard aux problèmes techniques soulevés par l'état de la technique considéré ci-dessus, un des objectifs essentiels de la présente invention est de fournir un article en particulier pour l'analyse biologique, comportant un emballage et un dispositif d'analyse à l'intérieur, dont l'ouverture de l'emballage soit facile, que le dispositif d'analyse soit rigide ou souple ; une telle ouverture pouvant se faire à une seule main.

Un autre objectif de la présente invention est de fournir un article en particulier pour l'analyse biologique dont l'ouverture de l'emballage n'entraîne pas de risque de blessure pour le manipulateur.

Un autre objectif de la présente invention est un article en particulier pour l'analyse biologique permettant une préhension aisée du dispositif d'analyse, une fois l'emballage ouvert.

Ces objectifs parmi d'autres sont résolus par la présente invention, qui concerne en premier lieu un article, en particulier pour l'analyse biologique, ledit article comprenant :
- une première feuille,
- une deuxième feuille, superposée à ladite première feuille,
   lesdites première et deuxième feuilles étant solidarisées sur l'ensemble de leurs côtés afin de former un emballage clos,
- au moins un dispositif d'analyse, disposé à l'intérieur dudit emballage clos ;
- un moyen de dessiccation rigide disposé à l'intérieur dudit emballage et comprenant une extrémité libre.

L'article selon l'invention est caractérisé en ce que la première feuille comporte une échancrure constituant une zone de pliure préférentielle, ladite échancrure étant en contact du moyen de dessiccation lors du pliage de l'emballage, et étant configurée pour exercer une pression sur ledit moyen de dessiccation de sorte que l'extrémité libre du moyen de dessiccation constitue un moyen de perforation de la deuxième feuille entraînant l'ouverture de l'emballage, lors du pliage de l'emballage.

Dans un mode de réalisation avantageux de l'article selon l'invention, le moyen de dessiccation est un matériau polymère solide renfermant un agent dessiccateur.

De façon préférentielle, le moyen de dessiccation a sensiblement la forme d'un parallélépipède rectangle. Une telle forme est particulièrement adaptée pour optimiser la perforation de l'emballage.

Selon un mode de réalisation particulier, l'une des desdites première et deuxième feuilles comporte une zone de pliure préférentielle.

Selon un autre mode de réalisation particulier, l'une des desdites première et deuxième feuilles comporte une zone de préhension du dispositif d'analyse.

Le dispositif d'analyse biologique contenu dans l'emballage peut avantageusement être une bandelette réactionnelle.

De façon préférentielle, la bandelette réactionnelle porte des antibiotiques sous forme d'un gradient de concentration.

Selon un autre mode de réalisation particulier, au moins l'une desdites première et deuxième feuilles comprend une couche d'aluminium.

Avantageusement, l'une desdites première et deuxième feuilles comprend un laminé composite aluminium - polymère.

Un autre objet de la présente invention concerne l'utilisation d'un article selon l'invention, pour l'analyse biologique.

Les buts et avantages de la présente invention seront mieux compris à la lumière de la description détaillée qui suit, faite en référence aux dessins dans lesquels :
La figure 1 représente une vue de dessus d'un article pour l'analyse biologique selon l'invention.
La figure 2 représente une vue en coupe longitudinale selon l'axe A-A de l'article pour l'analyse biologique représenté à la figure 1.
La figure 3 représente en coupe longitudinale selon l'axe A-A de l'article pour l'analyse biologique représenté à la figure 1, après pliage et ouverture de l'emballage.
La figure 4A représente une vue en perspective de l'article pour l'analyse biologique selon l'invention après pliage et ouverture de l'emballage.
La figure 4B représente une vue de dessus de l'article pour l'analyse biologique selon l'invention après pliage complet.

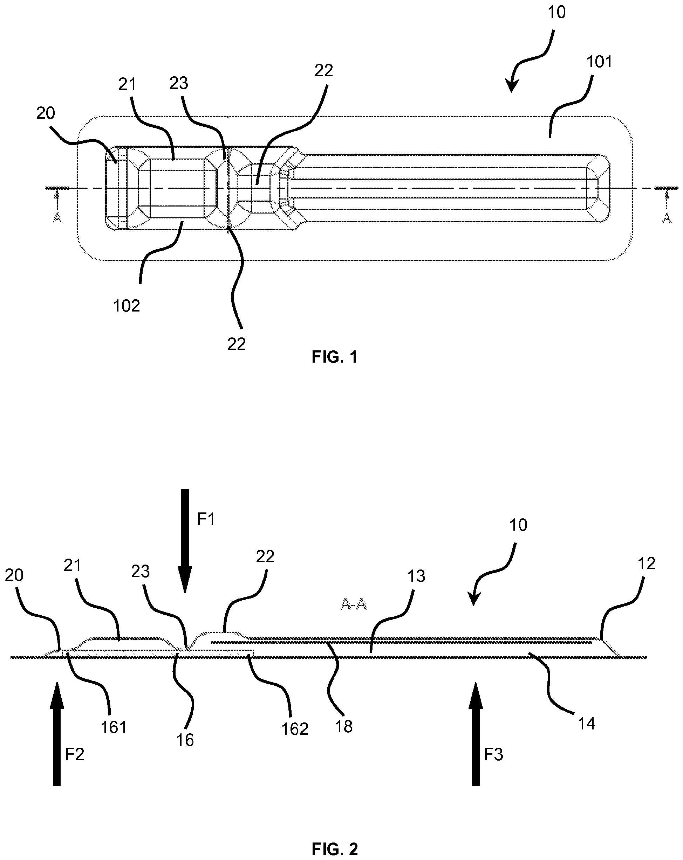

L'article 10 selon l'invention est représenté en vue de dessus sur la figure 1. Cet article est de forme générale sensiblement rectangulaire, avec une partie sensiblement plane 101 et une partie sensiblement bombée 102. Comme on peut le voir sur la figure 2, qui représente une coupe transversale de l'article 10 représenté sur la figure 1, la partie plane 101 constitue en fait une zone périphérique au niveau de laquelle la première feuille 12 et la deuxième feuille 14 constituant l'emballage sont solidarisées. Cette solidarisation des feuilles 12 et 14 est réalisée par thermo-scellage. La partie bombée 102 définit quant à elle, la cavité interne 13 de l'emballage. Il apparaît clairement sur la figure 2, que cette partie bombée 102 est essentiellement constituée par un moulage de la première feuille 12, constituant la feuille supérieure sur cette figure 2. A cette fin, le première feuille 12 est préférentiellement constituée d'un matériau souple, imperméable à l'eau. Un tel film peut être un laminé composite aluminium - plastiques (polyamide orienté (oPA), Polychlorure de vinyle (PVC)), ayant la capacité d'être mis en forme à froid et présentant une résistance minimale à la rupture. De tels films sont bien connus et largement utilisés dans l'emballage en particulier de médicaments, appelé communément blister. A titre d'exemple, on peut citer les films commercialisés par la société AMCOR sous la marque FORMPACK^{®}.

La feuille 14, constituant la feuille inférieure sur la figure 2, est quant à elle sensiblement plane. Il s'agit d'une feuille d'operculage. Elle doit par ailleurs présenter des propriétés de résistance à la rupture réduite. En effet, cette feuille a pour vocation d'être percée pour permettre d'accéder au dispositif d'analyse biologique 18, contenu dans l'emballage. Une telle feuille est avantageusement une feuille en aluminium, présentant des propriétés de scellage, notamment par adjonction sur l'aluminium d'une couche de polymère apte à permettre ce scellage. A titre d'exemple, on peut citer les films commercialisés par la société AMCOR sous la référence DD133

A l'intérieur de la cavité 13, sont présents un moyen de dessiccation 16 et un dispositif d'analyse 18. Le moyen de dessiccation 16 a pour fonction d'absorber l'humidité éventuellement emprisonnée dans la cavité 13 lors du scellage des feuilles 12 et 14. Il est ici constitué d'un matériau polymère solide renfermant un agent dessiccant. Un tel matériau est par exemple celui commercialisé par la société CSP Technologies^{™} sous la marque Activ-Polymer^{®}. Il est par ailleurs décrit dans le document WO-A-00/63288.

Le fait que le matériau constituant le moyen de dessiccation 16 se présente sous cette forme est essentiel dans la présente invention. En effet, il permet ainsi l'utilisation dudit moyen de dessiccation 16 comme moyen de perforation de l'emballage, tel que décrit *infra.* A cette fin, le moyen de dessiccation est préférentiellement de forme généralement sensiblement parallélépipédique rectangle. En effet, il apparaît que cette forme permet une ouverture optimisée de l'emballage, notamment par le fait que l'ouverture ainsi ménagée est relativement large permettant d'accéder facilement au dispositif d'analyse 18.

Pour ce qui concerne le dispositif d'analyse biologique 18, celui-ci peut prendre la forme d'une bandelette réactionnelle. A titre d'exemple, une telle bandelette réactionnelle peut être une bandelette portant des antibiotiques sous forme d'un gradient de concentrations. Une telle bandelette est commercialisée par la demanderesse sous la marque Etest^{®}.

Il est par ailleurs à noter que, si dans le mode de réalisation de l'article selon l'invention, décrit ici en lien avec les figures 1 à 4A, le dispositif se présente sous la forme d'une bandelette unique, il peut néanmoins tout à fait être envisageable de disposer plusieurs bandelettes superposées à l'intérieur de l'emballage.

Pour en revenir à la première feuille 12, celle-ci comme explicité *supra* présente une forme particulière. Ceci est bien visible sur la figure 2. En effet, elle présente une partie gauche avec des reliefs, alors que sa partie droite est quant à elle, relativement plane.

Concernant la partie gauche, il est tout d'abord à noter qu'elle présente une zone 20 avec un bossage limité. Il en résulte qu'à cet endroit, l'espace entre les feuilles 12 et 14 est relativement limité. Cette zone permet un positionnement très ajusté du moyen de dessiccation 16. En effet, dans cette zone, l'extrémité 161 du moyen de dessiccation se retrouve coincée entre les feuilles 12 et 14. Il est alors possible de maintenir le moyen de dessiccation en place, sans utilisation de moyen de collage quel qu'il soit, et notamment un adhésif.

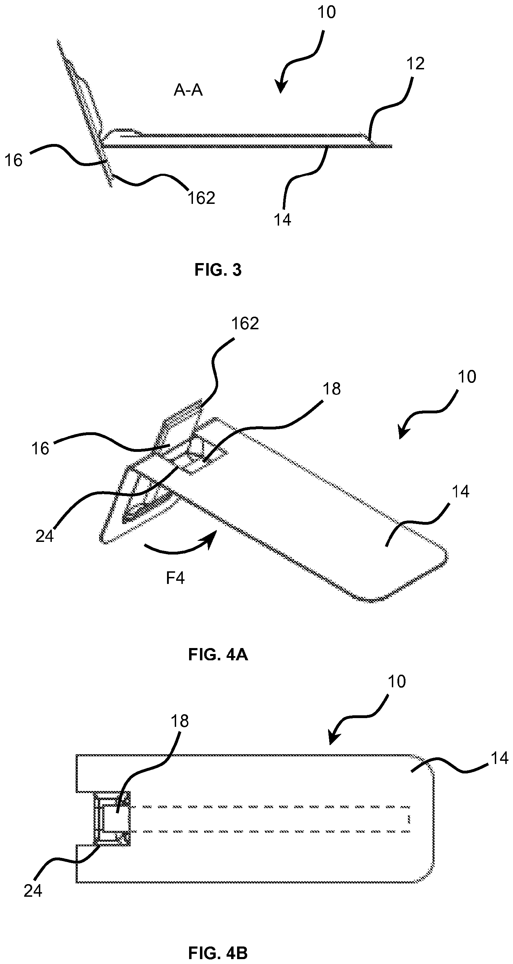

La partie gauche, en relief, comporte ensuite deux zones de bombage plus accentuées 21 et 22, séparées par une échancrure 23 sensiblement rectiligne et transversale. Cette disposition permet de concentrer les efforts au niveau de l'échancrure 23, lorsque l'emballage est plié pour permettre son ouverture. Les forces de pliage exercées sur l'emballage sont représentées sur la figure par les flèches F1, F2 et E3. La concentration des forces d'action au niveau de l'échancrure 23, entraine une pliure transversale à ce niveau. Ceci est bien représenté sur les figures 3 et 4A.

Par ailleurs, l'échancrure 23 présente une seconde fonction. Lors du pliage de l'emballage tel qu'explicité ci-dessus, l'échancrure 23 qui est au contact du moyen de dessiccation 16, joue le rôle d'un bras de levier exerçant une pression sur ledit moyen de dessiccation. Cette pression est répercutée sur l'extrémité libre 162 dudit moyen de dessiccation 16 qui vient alors percer la seconde feuille 16, entraînant ainsi l'ouverture de l'emballage.

Comme on peut le voir sur les figures 4A et 4B, sur lesquelles l'article 10 est représenté à l'envers par rapport à la figure 3, l'ouverture telle qu'elle est ménagée dans la feuille 14 fait apparaître le dispositif d'analyse biologique 18 et permet au manipulateur manipulant l'article pour l'analyse biologique 10 d'accéder audit dispositif 18. Pour ce faire, il lui suffit de replier totalement l'article 10 sur lui-même, conformément à la flèche F4, de sorte que les parties de la feuille 12 se trouvant de part et d'autre de la pliure, se retrouvent accolées. Cette configuration, représentée sur la figure 4B, permet un accès optimisé et facilité au dispositif d'analyse biologique 18, qui peut alors être extrait de l'emballage à l'aide d'un moyen de préhension, tel qu'une pince. A cet effet, la zone de bombage 22 permet de faciliter l'extraction du dispositif d'analyse biologique 18. En effet, comme on peut le voir sur les figures 2 et 3, cette zone de bombage 22 libère de manière substantielle l'espace autour de l'extrémité libre du dispositif d'analyse biologique 18, ce qui en facilite l'accès par le moyen de préhension.

## Revendications

1. Article (10), en particulier pour l'analyse biologique, ledit article comprenant :
- une première feuille (12),
- une deuxième feuille (14), superposée à ladite première feuille (12), lesdites première et deuxième feuilles étant solidarisées sur l'ensemble de leurs côtés afin de former un emballage clos,
- au moins un dispositif d'analyse (18), disposé à l'intérieur dudit emballage ;
- un moyen de dessiccation (16) rigide disposé à l'intérieur dudit emballage et comprenant une extrémité libre (162),
**caractérisé en ce que** la première feuille (12) comporte une échancrure (23) constituant une zone de pliure préférentielle, ladite échancrure (23) étant en contact du moyen de dessiccation (16) lors du pliage de l'emballage, et étant configurée pour exercer une pression sur ledit moyen de dessiccation (16) de sorte que l'extrémité libre (162) du moyen de dessiccation (16) constitue un moyen de perforation de la deuxième feuille (14) entraînant l'ouverture de l'emballage, lors du pliage de l'emballage.

2. Article (10) selon la revendication précédente, dans lequel le moyen de dessiccation (16) est un matériau polymère solide renfermant un agent dessiccateur.

3. Article (10) selon l'une des revendications précédentes, dans lequel le moyen de dessiccation (16) a sensiblement la forme d'un parallélépipède rectangle.

4. Article (10) selon l'une des revendications précédentes, dans lequel l'une des desdites première et deuxième feuilles (12, 14) comporte une zone de préhension du dispositif d'analyse.

5. Article (10) selon l'une des revendications précédentes, dans lequel le dispositif d'analyse biologique (18) est une bandelette réactionnelle.

6. Article (10) selon la revendication précédente, dans lequel la bandelette réactionnelle porte des antibiotiques sous forme d'un gradient de concentration.

7. Article (10) selon l'une des revendications précédentes, dans lequel au moins l'une des desdites première et deuxième feuilles (12, 14) comprend d'une couche d'aluminium.

8. Article (10) selon l'une des revendications précédentes, dans lequel au moins l'une des desdites première et deuxième feuilles (12, 14) comprend un laminé composite aluminium - polymère.

9. Utilisation d'un article (10) selon l'une des revendication 1 à 8, pour l'analyse biologique.

## Patentansprüche

1. Artikel (10), insbesondere zur biologischen Analyse, wobei der Artikel umfasst:
- eine erste Folie (12),
- eine zweite Folie (14), die über der ersten Bogen (12) liegt, wobei die ersten und zweiten Folien an allen ihren Seiten fest verbunden sind, um eine geschlossene Verpackung zu bilden,
- mindestens eine Analysevorrichtung (18), die im Inneren der Verpackung angeordnet ist;
- ein starres Trocknungsmittel (16), das im Inneren der Verpackung angeordnet ist und ein freies Ende (162) umfasst,
**dadurch gekennzeichnet, dass** die erste Folie (12) eine Einbuchtung (23) aufweist, die einen bevorzugten Knickbereich darstellt, wobei die Einbuchtung (23) beim Knicken der Verpackung mit dem Trocknungsmittel (16) in Kontakt ist und dazu ausgestaltet ist, einen Druck auf das Trocknungsmittel (16) auszuüben, so dass das freie Ende (162) des Trocknungsmittels (16) ein Mittel zur Perforation der zweiten Folie (14) darstellt, das beim Knicken der Verpackung zum Öffnen der Verpackung führt.

2. Artikel (10) nach dem vorhergehenden Anspruch, bei dem das Trocknungsmittel (16) ein festes Polymermaterial ist, das einen Trocknungsstoff umschließt.

3. Artikel (10) nach einem der vorhergehenden Ansprüche, bei dem das Trocknungsmittel (16) im Wesentlichen die Form eines Quaders hat.

4. Artikel (10) nach einem der vorhergehenden Ansprüche, bei dem eine der ersten und zweiten Folien (12, 14) einen Greifbereich der Analysevorrichtung aufweist.

5. Artikel (10) nach einem der vorhergehenden Ansprüche, bei dem die Vorrichtung zur biologischen Analyse (18) ein Reaktionsstreifen ist.

6. Artikel (10) nach dem vorhergehenden Anspruch, bei dem der Reaktionsstreifen Antibiotika in Form eines Konzentrationsgradienten trägt.

7. Artikel (10) nach einem der vorhergehenden Ansprüche, bei dem mindestens eine der ersten und zweiten Folien (12, 14) eine Aluminiumschicht umfasst.

8. Artikel (10) nach einem der vorhergehenden Ansprüche, bei dem mindestens eine der ersten und zweiten Folien (12, 14) ein Aluminium/Polymer-Verbundlaminat umfasst.

9. Verwendung eines Artikels (10) nach einem der Ansprüche 1 bis 8 zur biologischen Analyse.

## Claims

1. Article (10), in particular for biological analysis, said article comprising:
- a first sheet (12),
- a second sheet (14), superposed on said first sheet (12), said first and second sheets being joined along all of their sides in order to form a closed packaging,
- at least one analysis device (18), disposed inside said packaging;
- a rigid desiccating means (16) disposed inside said packaging and comprising a free end (162),
**characterized in that** the first sheet (12) has an indentation (23) constituting a preferential fold zone, said indentation (23) being in contact with the desiccating means (16) during folding of the packaging, and being configured to exert pressure on said desiccating means (16) such that the free end (162) of the desiccating means (16) constitutes a means for perforating the second sheet (14), causing the opening of the packaging, during folding of the packaging.

2. Article (10) according to the preceding claim, wherein the desiccation means (16) is a solid polymer material enclosing a desiccating agent.

3. Article (10) according to either of the preceding claims, wherein the desiccating means (16) substantially has the shape of a rectangular parallelepiped.

4. Article (10) according to one of the preceding claims, wherein one of said first and second sheets (12, 14) has a zone for gripping the analysis device.

5. Article (10) according to one of the preceding claims, wherein the biological analysis device (18) is a reaction strip.

6. Article (10) according to the preceding claim, wherein the reaction strip bears antibiotics in the form of a concentration gradient.

7. Article (10) according to one of the preceding claims, wherein at least one of said first and second sheets (12, 14) comprises a layer of aluminium.

8. Article (10) according to one of the preceding claims, wherein at least one of said first and second sheets (12, 14) comprises an aluminium-polymer composite laminate.

9. Use of an article (10) according to one of Claims 1 to 8, for biological analysis.
